# EUROPÄISCHE PATENTSCHRIFT

(11) **EP 3 755 394 B1**
(45) Veröffentlichungstag und Bekanntmachung des Hinweises auf die Patenterteilung: **23.11.2022**
(21) Anmeldenummer: 19705496.8
(22) Anmeldetag: 14.02.2019
(51) Int. Cl.: A61M 1/16, A61J 1/10, A61J 1/14

(54) **VORRICHTUNG ENTHALTEND EINE DIALYSELÖSUNG**
DEVICE CONTAINING A DIALYSIS FLUID
DISPOSITIF CONTENANT UN LIQUIDE DE DIALYSE

(30) Priorität: 21.02.2018 DE 102018103866
(43) Veröffentlichungstag der Anmeldung: 30.12.2020
(73) Patentinhaber: Fresenius Medical Care Deutschland GmbH, 61352 Bad Homburg (DE)
(72) Erfinder: WABEL, Peter, Dr., 61191 Rosbach (DE); BERLICH, Robert, Dr., 66606 St. Wendel (DE); BREUNINGER, Marcus, Dr., 61352 Bad Homburg (DE); STAUDE, Birgit, 64319 Pfungstadt (DE); RAU, Matthias, 65195 Wiesbaden (DE); WEISS, Stefan, Dr., 61352 Bad Homburg (DE); CERMAN, Zdenek, Dr., 65126 Idstein (DE)
(74) Vertreter: Herrmann, Uwe
(86) Internationale Anmeldenummer: PCT/EP2019/053676
(87) Internationale Veröffentlichungsnummer: WO 2019/162185

(56) Entgegenhaltungen:
- WO-A1-97/45149
- DE-B4- 19 825 568
- US-A- 4 326 526

## Beschreibung

Die Erfindung betrifft eine Vorrichtung zum Transport einer darin enthaltenen Dialyselösung.

Insbesondere im Bereich der Peritonealdialyse ist es üblich, dem Patienten Lösungsbeutel zur Verfügung zu stellen, die mit einer für den Patienten geeigneten Dialyselösung befüllt sind. Diese Lösungsbeutel schließt der Patient dann selbsttätig oder mit Unterstützung von medizinischem Fachpersonal an einen Einlaufschlauch, d.h. an den Patientenkatheter an, um das Peritoneum mit der Lösung zu füllen. Die Dialyselösung muss typischerweise in fertig zubereitetem Zustand zum Haus des Patienten transportiert werden. Aus der DE19825568A1 ist beispielsweise ein Folienbeutel mit Dialysierflüssigkeit bekannt. Weiterhin offenbart die US4326526A einen Beutel für Dialysat. Darüber hinaus offenbart die WO9745149 ein System zum Verpacken von Dialysezubehör.

Aufgabe der Erfindung ist es, eine Vorrichtung zum Transport einer Dialyselösung bereitzustellen, mit der ein sicherer Transport bei möglichst geringen Kosten und Abfallerzeugung gewährleistet werden kann.

Vor diesem Hintergrund betrifft die Erfindung eine Vorrichtung enthaltend eine Dialyselösung und einen medizinischen Konnektor zum Anschluss der Vorrichtung an eine zu einem Patienten führende Leitung, die einen flexiblen Innenbeutel zur Aufnahme der Dialyselösung aufweist, der in einer starren Umverpackung angeordnet ist. Bei Verwendung der Vorrichtung ist innerhalb des Innenbeutels eine Dialyselösung aufgenommen. Bei der Umverpackung handelt es sich vorzugsweise um eine quaderförmige Box.

Eine derartige Bag-In-Box-Verpackung hat den Vorteil, dass der typischerweise als Disposable ausgeführte Innenbeutel durch die Umverpackung vor mechanischen Einflüssen geschützt wird. Die mechanische Robustheit des Lösungsbeutels, hier Innenbeutels verliert gegenüber vorbekannten Lösungen an Bedeutung, wodurch eine materialsparende und kostengünstigere Ausführung ermöglicht wird. Insbesondere aufgrund der Tatsache, dass es sich bei Lösungsbeuteln typischerweise um Einwegprodukte handelt, ist die Kostenersparnis und die Abfallersparnis erheblich. Die Umverpackung kann aufwändiger gestaltet werden und gegebenenfalls auch mit Zusatzfunktionen wie Speichern oder patientenspezifischen Labels versehen werden. Aufgrund der Möglichkeit der vielfachen Verwendung spielen die Herstellungskosten und der Materialaufwand hier eine weniger große Rolle. Andererseits kann die Umverpackung beispielsweise kostensparend und umweltschonend aus Karton hergestellt werden. Die Umverpackung kann auch so ausgebildet sein, dass ein Stapeln der Vorrichtungen optimiert werden kann.

Der Innenbeutel ist typischerweise aus KunststofFfolien gefertigt, die entlang von Schweißnähten miteinander verbunden sind. Da Dialyselösungen oftmals einen Bicarbonatpuffer enthalten, werden vorzugsweise mehrlagige Folien mit einer geringen Gasdurchlässigkeit verwendet.

Die vorzugsweise quaderförmige Umverpackung kann aus einem starren Kunststoffmaterial, aus Metall oder aus Karton gefertigt sein.

In einer Ausführungsform ist vorgesehen, dass der Innenbeutel an wenigstens einer und vorzugsweise mehreren Innenwänden der Umverpackung befestigt ist, wobei vorzugsweise vorgesehen ist, dass an der oder den Innenwänden und dem Beutel korrespondierende Befestigungselemente vorgesehen sind, die reversibel lösbar miteinander verbunden werden können. Durch eine derartige Befestigung kann die Lage des Beutels in der Umverpackung definiert und stabilisiert werden. Beispiele umfassen mechanische Verbindungen wie Rastverbindungen, Steckelemente mit Vorsprüngen am Beutel und korrespondierende Durchbrüche oder Führungen an der Umverpackung sowie korrespondierende Haken und Aufnahmen. Neben den reversibel lösbaren Befestigungen kommen auch Befestigungen in Frage, die nicht zerstörungsfrei gelöst werden können, wie beispielsweise ein Verkleben oder Verschweißen.

In einer Ausführungsform ist vorgesehen, dass der Innenbeutel wenigstens einen Entnahmeanschluss zur Entnahme von Dialyselösung aufweist, der den medizinischen Konnektor umfasst, wobei vorzugsweise vorgesehen ist, dass die Umverpackung eine Entnahmeöffnung oder Entnahmeperforation aufweist, wobei der Entnahmeanschluss und die Entnahmeöffnung oder Entnahmeperforation so relativ zueinander angeordnet sind, dass durch die Entnahmeöffnung oder Entnahmeperforation auf den Entnahmeanschluss zugegriffen werden kann. Beispielsweise kann der Innenbeutel so in der Umverpackung angeordnet und befestigt sein, dass der Entnahmeanschluss direkt hinter oder in der Entnahmeöffnung oder Entnahmeperforation angeordnet ist. Der Innenbeutel und die Umverpackung können auch im Bereich des Entnahmeanschlusses und der Entnahmeöffnung oder Entnahmeperforation miteinander verbunden sein, um die Position des Entnahmeanschlusses relativ zur Entnahmeöffnung oder Entnahmeperforation zu fixieren. Die Entnahmeöffnung selbst kann beispielsweise anhand einer abziehbaren Folie oder eines Klappdeckels verschlossen sein, um den Entnahmeanschluss steril zu halten. Alternativ oder zusätzlich kann der Anschluss mit einer abnehmbaren Kappe oder einem abnehmbaren Überzug vorzugsweise steril verschlossen sein.

In einer Ausführungsform ist vorgesehen, dass der Innenbeutel so in der Umverpackung angeordnet ist, dass der Entnahmeanschluss in zumindest einer möglichen Standposition der Umverpackung an der tiefsten Stelle des Beutels angeordnet ist. So kann sichergestellt werden, dass Flüssigkeit vollständig aus dem Innenbeutel abfließen kann. Beispielsweise kann in der quaderförmigen Umverpackung an zumindest einer Seitenfläche eine Rampe angeordnet sein, die schräg zur jeweiligen Seitenfläche verläuft. So kann der Beutel schräg auf der Rampe aufliegen. Der Entnahmeanschluss kann am seitenflächennahen Ende der Rampe angeordnet sein. Alternativ kann der Beutel so geformt sein dass eine Seite des Beutels, die gegenüber einer an der Innenseite der Umverpackung befestigten Seite des Beutels liegt, abschüssig ist. Der Entnahmeanschluss kann am Ende des Abschusses angeordnet sein.

In einer Ausführungsform ist vorgesehen, dass die Umverpackung an einer Ecke oder Seitenkante eine Einhängevorrichtung aufweist und dass der Innenbeutel so in der Umverpackung angeordnet ist, dass der Entnahmeanschluss an der gegenüberliegenden Ecke oder Seitenkante der Umverpackung liegt. Wird die Umverpackung anhand der Einhängevorrichtung an einem geeigneten Ständer aufgehängt, befindet sich der Entnahmeanschluss dann an der tiefsten Stelle, sodass Flüssigkeit vollständig aus dem Innenbeutel abfließen kann.

In einer Ausführungsform ist vorgesehen, dass der Innenbeutel wenigstens einen Befüllanschluss zu dessen Befüllung mit Dialyselösung aufweist, wobei vorzugsweise vorgesehen ist, dass die Umverpackung eine Befüllöffnung aufweist, wobei der Befüllanschluss und die Befüllöffnung so relativ zueinander angeordnet sind, dass durch die Befüllöffnung auf den Befüllanschluss zugegriffen werden kann. So wird ein Befüllen des bereits in der Umverpackung befindlichen Beutels ermöglicht. Beispielsweise kann der Innenbeutel so in der Umverpackung angeordnet und befestigt sein, dass der Befüllanschluss direkt hinter oder in der Befüllöffnung oder Entnahmeperforation angeordnet ist. Der innenbeutel und die Umverpackung können auch Im Bereich des Befüllanschlusses und der Befüllöffnung miteinander verbunden sein, um die Position des Befüllanschlusses relativ zur Befüllöffnung zu fixieren. Vorzugsweise ist eine derartige Fixierung durch eine Rastverbindung, Schweißverbindung oder Klebeverbindung realisiert.

in einer Ausführungsform weist der Innenbeutel mindestens zwei Anschlüsse auf, einen Befüllanschluss zu dessen Befüllung mit Dialyselösung und einen Entnahmeanschluss zur Entnahme von Dialyselösung. Der Befüllanschluss kann an einer dem Entnahmeanschluss gegenüberliegenden Seite der Vorrichtung angeordnet sein.

In einer Ausführungsform ist vorgesehen, dass die Umverpackung wenigstens zwei Kompartimente umfasst, wobei der Innenbeutel in einem der beiden Kompartimente angeordnet ist. Das andere Kompartiment ist frei und kann zur Unterbringung von Schlauchsets oder anderen Zubehörteilen wie Handschuhen oder dergleichen genützt werden. Das Beutelkompartiment ist vorzugsweise so dimensioniert, dass es im Wesentlichen vollständig vom gefüllten Beutel ausgefüllt wird.

In einer alternativen Variante kann vorgesehen sein, dass der Innenbeutel die Umverpackung vollständig ausfüllt. Schlauchsets und Zubehörteile beispielsweise in einer Folienverpackung an der Außenseite der Umverpackung angeordnet sein.

In einer Ausführungsform ist vorgesehen, dass die Umverpackung einen Deckel aufweist, der eine Öffnung in den Innenraum der Umverpackung freigibt. Der Deckel kann beispielsweise so angeordnet sein, dass in ein Kompartiment des Innenraums zugegriffen werden kann, in dem der Beutel nicht angeordnet ist, sondern in dem ein Schlauchset und/oder Zubehörteile angeordnet sind. Der Deckel kann beispielsweise durch einen mit Perforationslinien getrennten Abschnitt der Umverpackung gebildet werden. Die Umverpackung kann so ausgebildet sein, dass der Innenraum vor einer Offnung des Deckels steril gegenüber der Umgebung abgeschlossen ist.

In einer Ausführungsform ist vorgesehen, dass die Vorrichtung ein Griffelement zum Tragen der Vorrichtung aufweist, wobei vorzugsweise vorgesehen ist, dass das Griffelement am Innenbeutel befestigt ist und durch eine Grifföffnung in der Umverpackung nach außen tritt. Durch eine derartige Anordnung wird die Kraft direkt in den Innenbeutel eingeleitet, der aufgrund seiner Füllung mit Dialyselösung typischerweise schwerer Ist als die Umverpackung.

In einer Ausführungsform ist vorgesehen, dass die Umverpackung so ausgebildet ist, dass sie durch ein Falten entlang vorgegebener Achsen in definierter Weise kollabiert werden kann. Beispielsweise kann die Box definierte Faltlinien oder Seitenflächen mit mehreren zueinander verklappbaren Flächen aufweisen. Durch ein Kollabieren kann von außerhalb der Umverpackung Druck auf den Innenbeutel ausgeübt werden, um dessen vollständige Entleerung zu begünstigen. Ferner ist ein kollabierter Behälter einfacher zu transportieren und hat im Falle einer Entsorgung ein geringeres Abfallvolumen.

In einer Ausführungsform ist vorgesehen, dass die Umverpackung ein Sichtfenster aufweist, um den Füllstand des Innenbeutels überwachen zu können. Dies kann beispielsweise dann von Vorteil sein, wenn die Vorrichtung nach dem Entleeren des Innenbeutels als Drainbag für gebrauchte Dialyselösung verwendet wird. Das Sichtfenster kann beispielsweise mit Füllstandsmarkierungen versehen sein.

In einer Ausführungsform ist vorgesehen, dass die Umverpackung gasdicht ausgeführt ist. Dadurch kann eine zusätzliche Gasbarriere geschaffen werden, was einerseits die Haltbarkeit bicarbonatgepufferter Dialyselösungen erhöht und andererseits eine dünnwandigere und mithin kosten- und materialsparendere Ausführung des Innenbeutels ermöglicht.

In einer Ausführungsform ist vorgesehen, dass der Behälter aus sterilisierbaren Materialien gefertigt ist. Vorzugsweise sind die Materialien so beschaffen, dass sie bei Temperaturen, die im Rahmen einer Hitzesterilisation einer Dialyselösung üblich sind, also beispielsweise etwa 120°C formstabil sind und sich nicht zersetzen oder Schadstoffe abgeben.

In einer Ausführungsform ist vorgesehen, dass alle gegenüber der Umgebung abgeschlossenen Innenräume des Behälters, die medizinische Flüssigkeiten oder Anschlüsse enthalten, steril sind. Dies gilt Insbesondere für das Innenvolumen des Innenbeutels und den medizinischen Konnektor.

In den Figuren zeigen:
- Figur 1:: eine erste Ausführungsform einer erfindungsgemäßen Transportbox In unterschiedlichen Verwendungsstadien;
- Figur 2:: Möglichkeiten zur Befestigung des Lösungsbeutels an der Umverpackung;
- Figur 3:: weitere Darstellungen der Box gemäß Figur 1;
- Figur 4:: eine alternative Variante der erfindungsgemäßen Transportbox;
- Figur 5:: eine mögliche Weiterbildung der Transportbox gemäß Figur 4;
- Figur 6:: eine weitere mögliche Weiterbildung der Transportbox gemäß Figur 4; und
- Figur 7:: eine kollabierbare Variante einer erfindungsgemäßen Box.

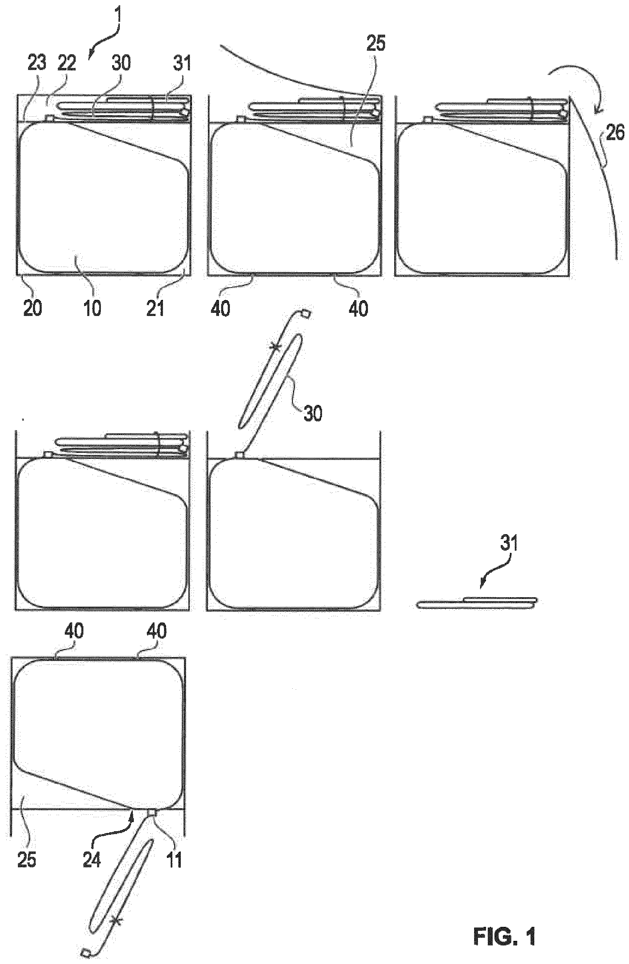

Eine Ausführungsform einer erfindungsgemäßen Transportbox ist in Figur 1 dargestellt. Die Transportbox 1 stellt eine Doppelverpackung nach Art einer Bag-in-Box-Verpackung für Dialyselösungen dar. Sie umfasst einen Innenbeutel 10 der mechanisch durch eine starre Umverpackung 20 gestützt und geschützt wird. Die Box 1 ist dafür bestimmt, in einem Behandlungszentrum oder in einem gegebenenfalls mobilen Ausgabeautomaten mit gebrauchsfertiger Dialyselösung befüllt und sodann vom Patienten nach Hause transportiert zu werden.

In der Abbildung links oben ist die Box 1 in dem Zustand dargestellt, in welcher sie an den Patienten übergeben wird. In diesem Zustand wird die Box 1 nun zunächst beschrieben.

Sowohl die quaderförmige Umverpackung 20 als auch der Innenbeutel 10 sind als Einwegprodukt konzipiert, wobei die Umverpackung aus Karton besteht und umweltschonen recycelt und sehr kostengünstig hergestellt werden kann. Der Innenbeutel 10 ist aus mehrlagigen Kunststofffolien gefertigt, die entlang von Schweißnähten miteinander verbunden sind. Die mechanische Robustheit des Innenbeutels 10 verliert gegenüber vorbekannten Lösungen jedoch an Bedeutung, wodurch eine materialsparende und kostengünstigere Ausführung des Innenbeutels 10 ermöglicht wird.

Die Umverpackung 20 umfasst zwei Kompartimente 21 und 22, die durch eine Zwischenwand 23 getrennt sind, wobei der Innenbeutel 10 in einem großen Beutelkompartiment 21 angeordnet ist. Im kleinen Schlauchkompartiment 22 ist ein Schlauchset 30 untergebracht, das mit weiteren Zubehörteilen 31 wie Handschuhen und dergleichen zu einem Bündel zusammengefasst ist. Das Beutelkompartiment 21 und der Innenbeutel 10 sind so dimensioniert, dass der Innenbeutel 10 das Beutelkompartiment 21 im Wesentlichen, d.h. bis auf eine rampenförmige Auslassung 25 vollständig ausfüllt. Die rampenförmige Auslassung 25 im Beutelkompartiment 21 ist in der gezeigten Ausführungsform eine Konsequent einer speziellen Formgebung des Innenbeutels 10 sowie Befestigung des Innenbeutels 10 an den Innenwänden des Beutelkompartiments 21. Als Alternative ist auch die Anordnung einer zur Umverpackung 20 gehörigen Rampe innerhalb des Beutelkompartiments denkbar. Die Funktion der rampenförmigen Auslassung 25 wird in weiterer Folge noch beschrieben werden.

Der Innenbeutel 10 weist einen Entnahmeanschluss 11 zur Entnahme von Dialyselösung auf. Der Entnahmeanschluss 11 des Innenbeutels 10 reicht durch eine Entnahmeöffnung 24 in der Zwischenwand 23 hindurch in das Schlauchkompartiment 22 hinein und ist mit dem Schlauchset 30 vorverbunden, sodass der Patient das Schlauchset 30 bei einer folgenden Anwendung nicht mehr an den Entnahmeanschluss 11 anzuschließen braucht. Obwohl dies in der Figur 1 nicht näher zu erkennen ist, sind der Innenbeutel 10 und die Umverpackung 20 im Bereich des Entnahmeanschlusses 11 und der Entnahmeöffnung 24 miteinander verbunden, um die Position des Entnahmeanschlusses 11 in der Entnahmeöffnung 24 zu fixieren.

Auch an der der Zwischenwand 23 gegenüberliegenden Wand des Beutelkompartiments 21 ist der Innenbeutel 10 an mehreren Stellen befestigt. Die entsprechenden Befestigungspunkte sind mit dem Bezugszeichen 40 gekennzeichnet.

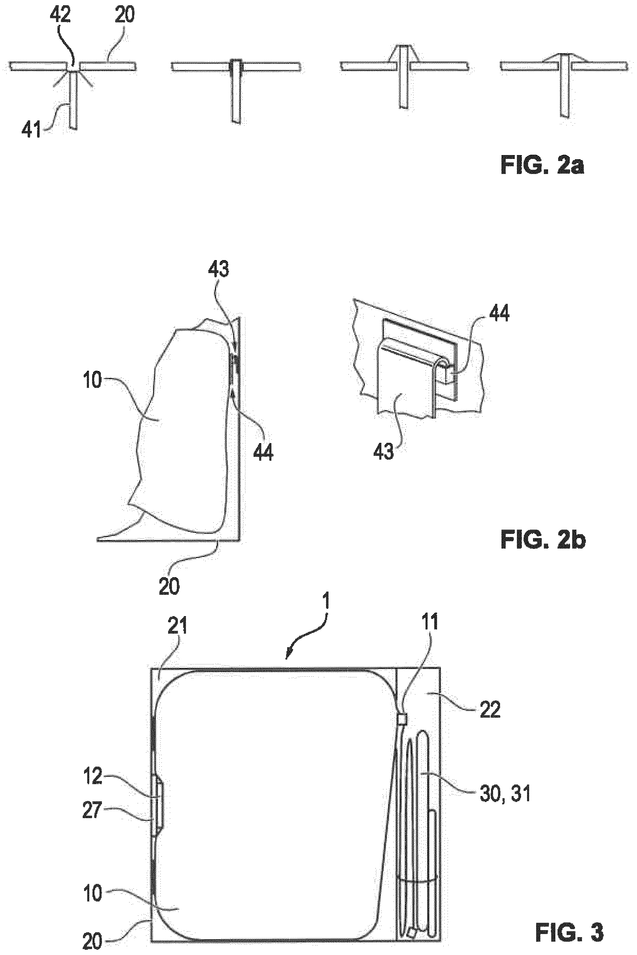

Die Befestigungen können beispielsweise als Klebepunkte oder Verschweißungen ausgebildet sein. Ferner Ist eine lösbare Ausführung denkbar, in welcher der Lösungsbeutel 10 mit Widerhaken 41 und die Wand der Umverpackung 20 mit Aufnahmelöchem 42 versehen ist, wie dies in der Figur 2a dargestellt ist.

Auch an den in Figur 1 seitlichen Wänden der Umverpackung 20 kann der Innenbeutel in einer Ausführungsform befestigt sein, um dessen Lage innerhalb der Umverpackung zu stabilisieren. Denkbar ist wiederum eine Befestigung anhand von Klebepunkte oder Verschweißungen oder eine lösbare Befestigung, beispielsweise nach Art wie in Figur 2b dargestellt, mit korrespondierenden Haltevorsprüngen 43 und 44 an Lösungsbeutel 10 bzw. Umverpackung 20.

Die Umverpackung 20 weist einen Deckel 26 auf, der das Schlauchkompartiment 22 an seiner der Zwischenwand 23 gegenüberliegenden Seite begrenzt. Der Deckel 26 erstreckt sich über die gesamte entsprechende Seite der Umverpackung 20 und besteht wie die verbleibenden Teile der Umverpackung 20 aus Karton, wobei er von den verbleibenden Teilen der Umverpackung 20 durch eine Perforationslinie getrennt ist.

Der Ablauf der Anwendung der Box 1 beim Patienten ist in den weiteren Abbildungen der Figur 1 schematisch erläutert.

Die stehende Box 1 der linken obigen Abbildung wird zunächst durch Aufreißen des Deckels 26 entlang der Perforationslinie und nachfolgendes Aufklappen des Deckels 26 geöffnet, sodass der Patient an das obenliegende Schlauchkompartiment 22 zugreifen kann. Sodann werden das mit dem Entnahmeanschluss 11 verbundene Schlauchset 30 und die Zubehörteile 31 aus dem Schlauchkompartiment 22 entnommen. Die Box wird dann um 180° gedreht, sodass der Entnahmeanschluss 11 unten liegt. In dieser Stellung kann die Box beispielsweise in erhöhter Stellung abgelegt oder an einem Ständer befestigt werden, dass sie an einer gegenüber dem Patienten erhöhten Position liegt. Ein Herausrutschen des Innenbeutels 10 aus der Umverpackung 20 wird in dieser Position durch die Befestigung an den Befestigungspunkten 40 verhindert. Durch die spezielle Formgebung des Innenbeutels 10, welche die rampenförmige Auslassung 25 freilässt, und durch dessen Fixierung an der nun obenliegenden Seite des Beutelkompartiments 21 liegt der Entnahmeanschluss an der tiefsten Stelle, sodass der Innenbeutel 10 bei der Behandlung gravimetrisch vollständig entleert werden kann.

Figur 3 zeigt ein weiteres Mal die bereits mit Blick auf Figur 1 geschriebene Box 1, wobei hier ein weiteres Detail der Box erkennbar wird, nämlich ein Befüllanschluss 12 an der dem Entnahmeanschluss 11 gegenüberliegenden Seite des Innenbeutels 10, d.h., an derjenigen Seite des Innenbeutels 10, welcher an den Befestigungspunkten 40 an der Innenwand des Beutelkompartiments 21 der Umverpackung 20 fixiert ist. An einer zum Befüllanschluss 12 korrespondierenden Stelle weist die Umverpackung 20 eine Befüllöffnung 27 auf, durch die von außen auf den Befüllanschluss 12 zugegriffen werden kann, sodass ein Befüllen des bereits in der Umverpackung 20 befindlichen Innenbeutels 10 ermöglicht wird.

Der Innenbeutel 10 weist also genau zwei Anschlüsse auf, nämlich den Befüllanschluss 11 zu dessen Befüllung mit Dialyselösung und den Entnahmeanschluss 12 zur Entnahme von Dialyselösung. Der Befüllanschluss 11 ist an der dem Entnahmeanschluss 12 gegenüberliegenden Seite des Innenbeutels 10 bzw. auch der Umverpackung 20 und der Box 1 insgesamt angeordnet.

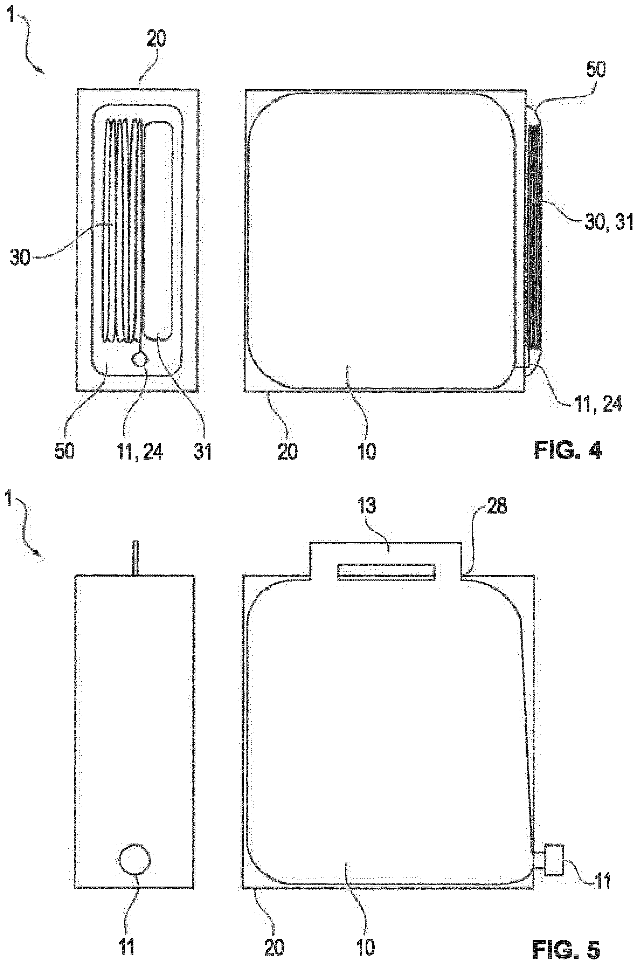

In Figur 4 ist eine alternative Variante der erfindungsgemäßen Box dargestellt, in welcher die Umverpackung 20 lediglich ein Kompartiment aufweist, das im Wesentlichen vollständig vom Innenbeutel 10 ausgefüllt wird. Ein Schlauchkompartiment fehlt in dieser Variante. Stattdessen sind das Schlauchset 30 und die Zubehörteile 31 in einer Folienverpackung 50 an der Außenseite der Umverpackung 20 aufgenommen. Die Entnahmeöffnung 24 der Umverpackung 20 ist unterhalb der Folienverpackung angeordnet und wird durch diese luftdicht überdeckt. Der Entnahmeanschluss 11 des Innenbeutels 10 reicht durch diese Entnahmeöffnung 24 hindurch in den Innenraum der Folienverpackung 50 und ist mit dem Schlauchset 30 vorverbunden, sodass der Patient bei einer folgenden Anwendung nur mehr die Folienverpackung öffnen muss und das Schlauchset 30 nicht mehr an den Entnahmeanschluss 11 anzuschließen braucht.

In Figur 5 ist eine weitere Variante einer erfindungsgemäßen Box 1 gezeigt, wobei ein Griffelement 13 zum Tragen der Box 1 am Innenbeutel 10 befestigt Ist und durch eine Grifföffnung 28 in der Umverpackung 20 nach außen tritt. Durch eine derartige Anordnung wird beim Tragen die Kraft direkt In den Innenbeutel 10 eingeleitet, der aufgrund seiner Füllung mit Dialyselösung typischerweise schwerer ist als die Umverpackung 20. Der Entnahmeanschluss 11 ist wie in der Figur 4 ausgebildet, wobei die Folienverpackung 50 nicht mehr gesondert dargestellt ist.

In der in Figur 4 dargestellten Variante einer erfindungsgemäßen Box 1 fehlt im Gegensatz zu der in Figur 1 dargestellten Variante die keilförmige Ausnehmung 25 im Innenraum der Außenverpackung 25, sodass dann, wenn die Box 1 flach am Boden aufgestellt Ist, der Innenbeutel 10 nicht zu seinem Entnahmeanschluss 11 hin zuläuft. Der Entnahmeanschluss 11 und die korrespondierende Entnahmeöffnung 24 sind aber im Bereich einer Seitenkante der Umverpackung 20 angeordnet, sodass der Innenbeutel bei einer schrägen Positionierung der Box 1 an der tiefsten Stelle des Innenbeutels angeordnet ist.

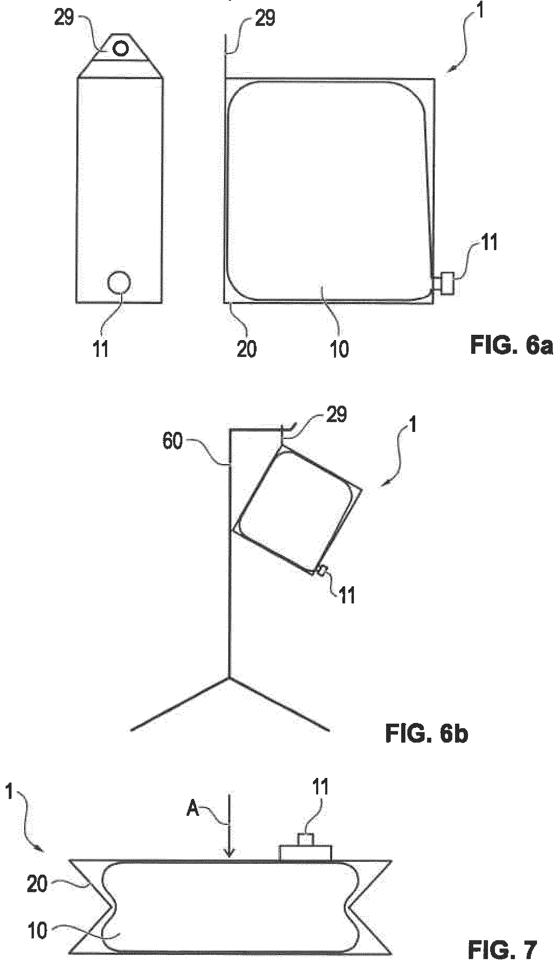

Deshalb kann die Box 1, wie dies in Figur 6a dargestellt ist, an der dem Entnahmeanschluss 11 und der Entnahmeöffnung 24 gegenüberliegenden Seitenkante der Umverpackung 20 eine Einhängelasche 29 aufweisen, mit welcher die Box 1, wie in Figur 6b dargestellt, an beispielsweise einem Infusionsständer 60 aufgehängt werden kann. Im eingehängten Zustand, liegt dann die Entnahmeöffnung 11, wie in Figur 6b erkennbar, automatisch an der tiefsten Stelle der Box, wodurch eine vollständige gravimetrische Entleerung des Innenbeutels 10 ermöglicht wird.

In Figur 7 ist eine Variante der erfindungsgemäßen Transportbox 1 dargestellt, in welcher die Umverpackung 20 so ausgebildet ist, dass sie durch ein Falten der Seitenwände entlang horizontaler Achsen kollabiert werden kann, sofern Druck in Richtung des Pfeils A auf die Oberseite der Box ausgeübt wird. Durch die Druckausübung kann ein vollständiges Entleeren des Innenbeutels 10 begünstigt werden, selbst wenn der Innenbeutel 10 nicht zur Entnahmeöffnung 11 hin zuläuft.

## Patentansprüche

1. Vorrichtung enthaltend eine Dialyselösung und einen medizinischen Konnektor zum Anschluss der Vorrichtung an eine zu einem Patienten führende Leitung,
**dadurch gekennzeichnet,**
**dass** die Vorrichtung einen flexiblen Innenbeutel zur Aufnahme der Dialyselösung aufweist, der in einer starren und vorzugsweise quaderförmigen Umverpackung angeordnet ist, wobei der Innenbeutel wenigstens einen Entnahmeanschluss zur Entnahme von Dialyselösung aufweist, der den medizinischen Konnektor umfasst, und wobei
die Umverpackung eine Einhängevorrichtung aufweist;
und **dass** der Innenbeutel so in der Umverpackung angeordnet ist, dass der Entnahmeanschluss an einer der Einhängevorrichtung gegenüberliegenden Ecke oder Seitenkante der Umverpackung liegt.

2. Vorrichtung nach Anspruch 1, **dadurch gekennzeichnet, dass** der Innenbeutel an wenigstens einer und vorzugsweise mehreren Innenwänden der Umverpackung befestigt ist, wobei vorzugsweise vorgesehen ist, dass an der oder den Innenwänden und dem Beutel korrespondierende Befestigungselemente vorgesehen sind, die reversibel lösbar miteinander verbunden werden können.

3. Vorrichtung nach einem der vorhergehenden Ansprüche, **dadurch gekennzeichnet, dass** vorgesehen ist, dass die Umverpackung eine Entnahmeöffnung oder Entnahmeperforation aufweist, wobei der Entnahmeanschluss und die Entnahmeöffnung oder Entnahmeperforation so relativ zueinander angeordnet sind, dass durch die Entnahmeöffnung oder Entnahmeperforation auf den Entnahmeanschluss zugegriffen werden kann.

4. Vorrichtung nach Anspruch 3, **dadurch gekennzeichnet, dass** der Innenbeutel so in der Umverpackung angeordnet ist, dass der Entnahmeanschluss in zumindest einer möglichen Standposition der Umverpackung an der tiefsten Stelle des Beutels angeordnet ist.

5. Vorrichtung nach Anspruch 3, **dadurch gekennzeichnet, dass** die Umverpackung die Einhängevorrichtung an einer Ecke oder Seitenkante aufweist.

6. Vorrichtung nach einem der vorhergehenden Ansprüche, **dadurch gekennzeichnet, dass** der Innenbeutel wenigstens einen Befüllanschluss zu dessen Befüllung mit Dialyselösung aufweist, wobei vorzugsweise vorgesehen ist, dass die Umverpackung eine Befüllöffnung aufweist, wobei der Befüllanschluss und die Befüllöffnung so relativ zueinander angeordnet sind, dass durch die Befüllöffnung auf den Befüllanschluss zugegriffen werden kann.

7. Vorrichtung nach einem der vorhergehenden Ansprüche, **dadurch gekennzeichnet, dass** die Umverpackung wenigstens zwei Kompartimente umfasst, wobei der Innenbeutel in einem der beiden Kompartimente angeordnet ist.

8. Vorrichtung nach einem der vorhergehenden Ansprüche, **dadurch gekennzeichnet, dass** die Umverpackung einen Deckel aufweist, der eine Öffnung in den Innenraum der Umverpackung freigibt.

9. Vorrichtung nach Anspruch 8, **dadurch gekennzeichnet, dass** der Innenraum vor einer Offnung des Deckels steril gegenüber der Umgebung abgeschlossen ist.

10. Vorrichtung nach einem der vorhergehenden Ansprüche, **dadurch gekennzeichnet, dass** die Vorrichtung ein Griffelement zum Tragen der Vorrichtung aufweist, wobei vorzugsweise vorgesehen ist, dass das Griffelement am Innenbeutel befestigt ist und durch eine Grifföffnung in der Umverpackung nach außen tritt.

11. Vorrichtung nach einem der vorhergehenden Ansprüche, **dadurch gekennzeichnet, dass** die Umverpackung so ausgebildet ist, dass sie durch ein Falten entlang vorgegebener Achsen in definierter Weise zusammengelegt werden kann.

12. Vorrichtung nach einem der vorhergehenden Ansprüche, **dadurch gekennzeichnet, dass** der Behälter aus sterilisierbaren Materialien gefertigt ist.

13. Vorrichtung nach einem der vorhergehenden Ansprüche, **dadurch gekennzeichnet, dass** alle gegenüber der Umgebung abgeschlossenen Innenräume des Behälters, die medizinische Flüssigkeiten oder Anschlüsse enthalten, steril sind.

## Claims

1. An apparatus containing a dialysis solution and a medical connector for connecting the apparatus to a line leading to the patient,
**characterized in that**
the apparatus has a flexible inner bag for receiving the dialysis solution that is arranged in a rigid outer packaging that is preferably parallelepiped-shaped, wherein the inner bag has at least one extraction port for the extraction of dialysis solution that comprises the medical connector and wherein
the outer packaging has a suspension apparatus;
and **in that** the inner bag is arranged in the outer packaging such that the extraction port is located at the corner or side edge of the outer packaging that is opposite the outer packaging.

2. An apparatus in accordance with claim 1, **characterized in that** the inner bag is fastened to at least one, and preferably to a plurality of inner walls of the outer packaging, with provision preferably being made that corresponding fastening elements that can be reversibly connected to one another are provided at the inner wall or walls and at the bag.

3. Apparatus in accordance with one of the preceding claims, **characterized in that** it is provided that the outer packaging has an extraction opening or an extraction perforation, with the extraction port and the extraction opening or extraction perforation being arranged relative to one another such that access can be made to the extraction port through the extraction opening or extraction perforation.

4. An apparatus in accordance with claim 3, **characterized in that** the inner bag is arranged in the outer packaging such that the extraction port is arranged in at least one possible standing position of the outer packaging at the lowest point of the bag.

5. An apparatus in accordance with claim 3, **characterized in that** the outer packaging has a suspension apparatus at a corner or side edge.

6. An apparatus in accordance with one of the preceding claims, **characterized in that** the inner bag has at least one filling port for its filling with dialysis solution, with provision preferably being made that the outer packaging has a filling opening, with the filling port and the filling opening being arranged relative to one another such that access can be made to the filling port through the filling opening.

7. An apparatus in accordance with one of the preceding claims, **characterized in that** the outer packaging comprises at least two compartments, with the inner bag being arranged in one of the two compartments.

8. An apparatus in accordance with one of the preceding claims, **characterized in that** the outer packaging has a cover that releases an opening into the inner space of the outer packaging.

9. An apparatus in accordance with claim 8, **characterized in that** the inner space is closed off in a sterile manner with respect to the environment prior to an opening of the cover.

10. An apparatus in accordance with one of the preceding claims, **characterized in that** the apparatus has a gripping element for carrying the apparatus, with provision preferably being made that the gripping element is fastened to the inner bag and passes to the outside through a gripping opening in the outer packaging.

11. An apparatus in accordance with one of the preceding claims, **characterized in that** the outer packaging is configured such that it can be folded together in a defined manner by a folding along predefined axes.

12. An apparatus in accordance with one of the preceding claims, **characterized in that** the container is produced from sterilizable materials.

13. An apparatus in accordance with one of the preceding claims, **characterized in that** all the inner spaces of the container containing medical liquids or ports that are closed off with respect to the environment are sterile.

## Revendications

1. Dispositif contenant une solution de dialyse et un connecteur médical pour le raccordement du dispositif à un conduit menant à un patient,
**caractérisé en ce**
**que** le dispositif présente un sachet intérieur flexible pour recevoir le liquide de dialyse, qui est disposé dans un emballage entourant rigide et de préférence de forme carrée, dans lequel le sachet intérieur présente au moins un raccord de prélèvement pour prélever de la solution de dialyse, qui comprend le connecteur médical, et dans lequel
l'emballage entourant présente un dispositif d'accrochage ;
et **que** le sachet intérieur est disposé de telle sorte dans l'emballage entourant que le raccord de prélèvement se situe sur un angle ou sur un bord latéral de l'emballage entourant faisant face au dispositif d'accrochage.

2. Dispositif selon la revendication 1, **caractérisé en ce que** le sachet intérieur est fixé sur au moins une et de préférence plusieurs parois intérieures de l'emballage entourant, dans lequel il est prévu de préférence que sont prévus sur la ou les parois intérieures et sur le sachet des éléments de fixation correspondants, qui peuvent être reliés les uns aux autres de manière amovible et réversible.

3. Dispositif selon l'une quelconque des revendications précédentes, **caractérisé en ce qu'**il est prévu que l'emballage entourant présente une ouverture de prélèvement ou une perforation de prélèvement, dans lequel le raccord de prélèvement et l'ouverture de prélèvement ou la perforation de prélèvement sont disposés les uns par rapport aux autres de telle sorte qu'il est possible de recourir au raccord de prélèvement par l'ouverture de prélèvement ou la perforation de prélèvement.

4. Dispositif selon la revendication 3, **caractérisé en ce que** le sachet intérieur est disposé de telle sorte dans l'emballage entourant que le raccord de prélèvement est disposé sur l'emplacement le plus bas du sachet dans au moins une position debout éventuelle de l'emballage entourant.

5. Dispositif selon la revendication 3, **caractérisé en ce que** l'emballage entourant présente le dispositif d'accrochage sur un angle ou un bord latéral.

6. Dispositif selon l'une quelconque des revendications précédentes, **caractérisé en ce que** le sachet intérieur présente au moins un raccord de remplissage aux fins de son remplissage avec de la solution de dialyse, dans lequel il est prévu de préférence que l'emballage entourant présente une ouverture de remplissage, dans lequel le raccord de remplissage et l'ouverture de remplissage sont disposés l'un par rapport à l'autre de telle sorte qu'il est possible de recourir au raccord de remplissage par l'ouverture de remplissage.

7. Dispositif selon l'une quelconque des revendications précédentes, **caractérisé en ce que** l'emballage entourant comprend au moins deux compartiments, dans lequel le sachet intérieur est disposé dans l'un des deux compartiments.

8. Dispositif selon l'une quelconque des revendications précédentes, **caractérisé en ce que** l'emballage entourant présente un couvercle, qui dégage une ouverture dans l'espace intérieur de l'emballage entourant.

9. Dispositif selon la revendication 8, **caractérisé en ce que** l'espace intérieur devant une ouverture du couvercle est fermé de manière stérile par rapport à l'environnement.

10. Dispositif selon l'une quelconque des revendications précédentes, **caractérisé en ce que** le dispositif présente un élément de préhension pour supporter le dispositif, dans lequel il est prévu de préférence que l'élément de préhension est fixé sur le sachet intérieur et se déplace vers l'extérieur par une ouverture de préhension dans l'emballage entourant.

11. Dispositif selon l'une quelconque des revendications précédentes, **caractérisé en ce que** l'emballage entourant est réalisé de telle sorte qu'il peut être replié d'une manière définie par un pliage le long d'axes prédéfinis.

12. Dispositif selon l'une quelconque des revendications précédentes, **caractérisé en ce que** le contenant est produit à partir de matériaux pouvant être stérilisés.

13. Dispositif selon l'une quelconque des revendications précédentes, **caractérisé en ce que** tous les espaces intérieurs du contenant fermés par rapport à l'environnement, qui contiennent des liquides ou des raccords médicaux, sont stériles.
